# EUROPEAN PATENT APPLICATION

(11) **EP 1 873 243 A1**
(43) Date of publication of application: **02.01.2008**
(21) Application number: 07252566.0
(22) Date of filing: 25.06.2007
(51) Int. Cl.: C12N 15/10

(54) **Isolation of RNA and DNA from a biological sample**

(30) Priority: 18.01.2007 US 881058 P; 29.06.2006 US 817504 P
(71) Applicant: MILLIPORE CORPORATION, Billerica, MA 01821-2405 (US)
(72) Inventor: Ongena, Kathleen, Reading, MA 01867 (US); Baggio, Ricky Francis, Waltham, MA 02453 (US)
(74) Representative: Kirkham, Nicholas Andrew

(57) **Abstract**

The invention relates to methods of isolating nucleic acids from a sample using a filter device comprising a plurality of membranes. The invention also provides for devices comprising a plurality of membranes and kits suitable for isolating nucleic acids from a sample.

SEQUENCE LISTING

<110>  MILLIPORE CORPORATION

<120>  ISOLATION OF RNA AND DNA FROM A BIOLOGICAL SAMPLE

<130>  P9773EP00

<160>  4

<170>  PatentIn version 3.4

<210>  1
<211>  20
<212>  DNA
<213>  ARTIFICIAL

<220>
<223>  ARTIFICIAL PRIMER

<400>  1
gtggggcgcc ccaggcacca                                                    20


<210>  2
<211>  21
<212>  DNA
<213>  ARTIFICIAL

<220>
<223>  ARTIFICIAL PRIMER

<400>  2
ctccttaatg tcacgcacga t                                                  21


<210>  3
<211>  20
<212>  DNA
<213>  ARTIFICIAL

<220>
<223>  ARTIFICIAL PRIMER

<400>  3
ggtctgagag gatgatcagt                                                    20


<210>  4
<211>  18
<212>  DNA
<213>  ARTIFICIAL

<220>
<223>  ARTIFICIAL PRIMER

<400>  4
ttagctccac ctcgcggc                                                      18

## Description

This application claims the benefit of U.S. Provisional Application No. 60/817,504 filed June 29, 2006 and U.S. Provisional Application No. 60/881,058 filed January 18, 2007 both of which are hereby incorporated by reference in their entirety.

### Field of the Invention

The invention relates generally to the field of molecular biology. In certain specific embodiments the invention provides devices, kits and methods relating to the isolation of nucleic acids.

### Background of the Invention

Isolating nucleic acids is typically the first step of most molecular biological inquiries including PCR, gene cloning, sequencing, Southern analysis, Northern analysis, nuclease protection assays, RT-PCR, RNA mapping, in vitro translation, in vitro transcription, including transcription/amplification reactions and cDNA library construction. Obtaining high quality intact nucleic acids suitable for analysis is thus often a useful and desirable starting point for subsequent analyses. A variety of techniques for isolating nucleic acids from a sample have been described (see, e.g., U.S. Patent Nos. 5,075,430; 5,234,809, 5,155,018; 6,277,648; 6,958,392; 6,953,686; 6,310,199; 6,992,182; 6,475,388; 5,075,430; 7,074,916; U.S. Patent Publication No. 20060024701; European Patent No. EP0765335; Boom et al. 1990, J. Clinical Microbiology 28:495). Many of these previously described methods relied on cesium chloride density gradient centrifugation or phenol extraction both of which had significant shortcomings including time consumption, exposure to hazard chemicals and high risk of contaminating samples with foreign nucleic acids or undesirable proteins. Other methods relied on silica based solid supports, but did not provide a means of isolating both DNA and RNA. It would thus be, beneficial to provide a method of isolating nucleic acids, from a sample that was fast, easy to perform, economical, and produced high yields. It would also be useful if the method minimized the required manipulation of the sample, permitted the use of primarily liquid handling steps and could be performed using a single device, e.g. a filtering device. It would be desirable to be able to isolate both DNA and RNA using a single device, e.g. a device comprising two membranes, and a single method wherein the method was comprised of relatively few steps compared to previously described nucleic acid purification protocols. Various embodiments of the invention described herein meet these and other needs.

### SUMMARY OF THE INVENTION

In certain embodiments the invention provides a method of isolating a first and second species of nucleic acid from a biological sample comprising contacting the biological sample with a first porous material, e.g. a membrane, and a second porous material, e.g. a membrane, such that the first species of nucleic acid binds to the first porous material and the second species of nucleic acid binds to the second porous material. The first and second porous materials may be washed with one or more suitable buffers after each of the contacting steps. The first and second nucleic acid species may be eluted from the first and second porous material by contacting each respective porous material with a suitable elution buffer.

In other embodiments the invention provides a method of isolating DNA and RNA from a cellular sample comprising a) lysing the cellular sample to obtain a cellular lysate; b) optionally clarifying the cellular lysate, e.g. by centrifugation,; c) contacting the lysate with a first porous material such that the RNA binds to the first porous material; and contacting the lysate, or a filtrate from the first membrane with a second porous material such that the DNA binds to the second porous material thereby separately isolating DNA and RNA from the cellular sample. The first porous material may have a nominal pore size that is smaller than the nominal pore size of the second porous material. The method may include washing the first and second porous material with one or more suitable buffers. The RNA and DNA may be eluted off of the first and second porous material respectively by contacting each respective porous material with a suitable elution buffer.

In still other embodiments the invention provides a method of isolating genomic DNA from cellular RNA from a cellular sample comprising a) lysing the cellular sample with a lysis buffer to obtain a cellular lysate; b) optionally clarifying the cellular sample, e.g. by centrifugation, to obtain a clarified supernatant; c) contacting the lysate with a first membrane, e.g., a polysaccharide membrane such that the RNA binds to the first membrane; and d) contacting the lysate or filtrate from the first membrane with a second membrane, e.g., a silicate membrane such that the DNA binds to the second membrane thereby isolating genomic DNA and RNA from the cellular sample. The method may include washing the first and second porous material with one or more suitable buffers. The RNA and DNA may be eluted off of the first and second porous material respectively by contacting each porous material with a suitable elution buffer.

In further embodiments the invention provides a method of isolating genomic DNA from cellular RNA from a eukaryotic cellular sample comprising a) lysing the cellular sample with a lysis buffer to obtain a cellular lysate; b) contacting the lysate with a first membrane, e.g., a polysaccharide membrane such that the RNA binds to the first membrane; and c) contacting the lysate or a filtrate from the first membrane with a second membrane, e.g., a silicate membrane such that the DNA binds to the second membrane thereby isolating genomic DNA and RNA from the cellular sample. The method may include washing the first and second porous material with one or more suitable buffers. The RNA and DNA may be eluted off of the first and second porous material respectively by contacting each porous material with a suitable elution buffer.

In yet further embodiments the invention provides a method of isolating genomic DNA from cellular RNA from a prokaryotic cellular sample comprising a) lysing the cellular sample with a lysis buffer to obtain a cellular lysate; b) clarifying the cellular sample, e.g. by centrifugation, to obtain a clarified supernatant; c) contacting the lysate with a first membrane, e.g., a polysaccharide membrane such that the RNA binds to the first membrane; and d) contacting the lysate or a filtrate from the first membrane with a second membrane, e.g., a silicate membrane such that the DNA binds to the second membrane thereby isolating genomic DNA and RNA from the cellular sample. The method may include washing the first and second porous material with one or more suitable buffers. The RNA and DNA may be eluted off of the first and second porous material respectively by contacting each porous material with a suitable elution buffer.

In certain other embodiments the invention provides a filter device comprising a silicate membrane and a polysaccharide membrane, such as a mixed cellulose ester (MCE) membrane suitable for isolating a first species of nucleic acid, and a second species of nucleic acid from a biological sample. The first nucleic acid species may be RNA which binds to the polysaccharide membrane e.g., a cellulose membrane and the second species may be DNA, e.g. genomic DNA, which binds to the silicate membrane, e.g., a glass fiber membrane.

In yet other embodiments the invention provides a kit for isolating a nucleic acid from a sample comprising a) a first porous material, e.g. a membrane comprising a polysaccharide and b) a second porous material, e.g., a membrane, comprising a silicate; c) optionally one or more wash buffers and b) at least one container. In still other embodiments the invention provides a kit for isolating RNA from a cellular sample comprising a) a mixed cellulose ester membrane; b) one or more non-acidic wash buffers; and c) one or more containers.

### BRIEF DESCRIPTION OF THE DRAWINGS

Figures 1 a and 1 b show one example of a filter device of the invention which is comprised of a micropartition device.
Figure 2 shows an example of a filter device of the invention.
Figures 3a and 3b show an example of a filter device of the invention in multiwell format suitable for receiving multiple samples or large volume samples.
Figure 4 shows another example of a filter device of the invention in multiwell format suitable for receiving multiple samples or large volume samples.
Figure 5 shows a single sample version of a filter device of the invention.
Figure 6a shows a closed version of a filter device of the invention: vented and non-vented.
Figure 6b shows a stacked version of a filter device of the invention.
Figure 7 shows a controllable, automated system with a closed, vented version of the filter device of the invention.
Figure 8 is a flow chart depicting one embodiment of the invention for isolating RNA and genomic DNA.
Figures 9a and 9b are photographs of an agarose gel showing isolation of genomic DNA and 16S and 23S RNA from a prokaryotic cellular sample.
Figures 10a and 10b are photographs of an agarose gel showing isolation of genomic DNA and 16S and 23S RNA from a prokaryotic cellular sample using two stacked 96 well plates comprising either a mixed cellulose ester membrane or a glass fiber membrane.
Figures 11a and 11b are photographs of an agarose gel showing isolation of genomic DNA and 18S and 28S RNA from a eukaryotic cellular sample.
Figures 12a and 12b are photographs of agarose gels showing rtPCR products using RNA obtained from a eukaryotic cell (12a) and a prokaryotic cell (12b) according to one embodiment of the invention.
Figure 13 is a photograph of an agarose gel showing the purity of RNA isolated according to one embodiment of the invention compared to a commercially available RNA purification column.

### DESCRIPTION OF THE EMBODIMENTS

### Methods of Isolating Nucleic Acids

In some embodiments the invention provides a method of isolating one or more species of nucleic acids from a sample which is fast, easy to perform and requires a minimal number of manipulations to the sample. The method offers the advantage of accepting cellular, e.g., prokaryotic or eukaryotic input in the form of a cellular lysate, and discharging a plurality of target molecules as product. The cellular lysate may be prepared before the lysate is contacted with the porous material according to the invention. Thus the invention provides a single method to isolate both DNA and RNA from a cellular extract.

The method is easy to perform in that it requires few steps and few reagents. The reagents are less toxic than reagents used in previously described methods, and the method yields a substantially pure product comprising DNA, e.g. genomic DNA and RNA, e.g. cellular RNA comprising mRNA, rRNA and tRNA. Substantially pure, as used herein means the product is substantially free of other biochemical species, e.g., at least 60% pure, at least 70% pure, at least 80% pure, at least 90% pure, at least 95% pure. Substantial purity may be determined for example by electrophoresing an aliquot of an isolated nucleic acid, such as an isolated DNA, or an isolated RNA, through an agarose gel. Intensity of the bands may be determined using known methods, e.g., using a gel reader comprising a spectrophotometer.

The easily performed method may include lysing a cellular sample. Lysing the cells may be performed using any lysis buffer known in the art. Suitable lysis buffers may be comprised of a chaotropic agent such as a guanidinium salt, e.g. guanidinium thio cyanate. The lysis buffer may comprise a chelating agent, e.g. EDTA and a buffering salt such as TRIS-HCl. In a specific embodiment the lysis buffer has a non-acidic pH. e.g. neutral or basic.

After lysis, the sample may be clarified, e.g. centrifuged to remove cellular debris and particulate matter to obtain a cellular a clarified lysate. The method may comprise contacting a plurality of porous materials such as a plurality of membranes with the supernatant. In a specific embodiment the cellular lysate is contacted first with a polysaccharide membrane such as a mixed cellulose ester membrane, or a nitrocellulose membrane followed by a silicate membrane, e.g. a glass fiber membrane. The membranes may be stacked one upon the other. For example the polysaccharide membrane may be stacked on top of the silicate membrane such that the lysate contacts the polysaccharide membrane first. The method thus allows the artisan to merely perform one step in isolating cellular DNA from cellular RNA, e.g., contacting the cellulose membrane with the supernatant and allowing gravity or an externally supplied force to permit the silicate membrane to be contacted subsequently. Where the membranes are stacked and it is desirable to elute both the DNA and RNA products from their respective membranes, the membranes may conveniently be separated prior to elution of the product. Alternatively, the membranes may be arranged in series such that the cellular lysate is first applied to the polysaccharide membrane and the filtrate from that membrane is next applied to the silicate membrane. Once the plurality of membranes has been contacted with the cellular lysate the membranes may be subjected to one or more washes. In certain embodiments where high product yield is desired the filtrate from on or more of the respective membranes may be recirculated over the first and second membranes one or more times.

Suitable wash buffers for use in the methods of the invention may include non-acidic buffers, e.g.,buffers having a neutral or basic pH. In a specific embodiment two distinct wash buffers may be used, e.g., a first and second wash buffer. The first wash buffer may be applied to the membranes before the second wash buffer and may comprise a chaotropic agent such as a guanidinium salt, e.g. guanidinium thio cyanate. The first wash buffer may contact the sample (cellular lysate) after it has contacted the first membrane. The first wash buffer may further comprise a chelating agent, e.g. EDTA and a buffering salt such as TRIS-HCl as well as an alcohol, e.g., ethanol. The second wash buffer may comprise an alcohol, e.g., ethanol and a buffering salt such as TRIS-HCl.

After washing the membranes the final product may be eluted with any suitable elution buffer, e.g. water, Tris-EDTA buffer (TE). A suitable buffer for eluting the RNA product may include an RNase free buffer such as RNase free water.

The isolated product may be suitable for further manipulation, e.g. PCR including RT-PCR, transcription mediated amplification, transfection, sequencing and the like. Isolation of the target nucleic acid may be used for many down stream analytical applications including, for example, identification of a pathogenic or non-pathogenic organism of interest, such as a bacterium. Where the target nucleic acid is RNA, the invention provides a method of isolating RNA that does not require the use of DNAse, or alternatively, requires the use of less DNAse than is required by previously described methods, e.g., Qiagen columns (Qiagen, Inc., Valencia, CA) thereby saving time and reagent cost.

In embodiments of the invention where the sample is applied to one or more membranes an external force may be applied to that membrane after the sample has contacted it to facilitate passage of the cellular lysate through the membrane and isolation of the target nucleic acid on the membrane. In some embodiments the force may be provided by gravity or by a vacuum attached to the filter device, e.g. via an outlet on the bottom of the device. When the external force is a vacuum, the vacuum may provide a pressure difference of 20-800 mbars. In other embodiments the force may be provide by elevated positive pressure from the top of the filtration device, e.g., by the action of a piston or member, where the device is comprised of a cylinder or a syringe. In still other embodiments the force may be a centrifugal force applied by placing the device in a centrifuge. Centrifugal forces ranging from 1×g -15,000×g may be used.

In certain embodiments the nucleic acid may be eluted off of the first or second membrane onto a third membrane. The third membrane may comprise a capture probe, e.g. a specific binding partner for the target nucleic acid such that the target nucleic acid is preferentially retained on the third membrane as a result of a specific chemical interaction between the target nucleic acid and the target probe. The capture probe may interact covalently or non-covalently with the target nucleic acid. The interaction may include charge-charge interactions, hydrogen bonds, hydrophobic interactions, van Der Waals forces and dipole-dipole interactions.

Examples of capture probes include oligos which are complementary to a particular nucleic acid sequence of interest, as well as peptides, proteins and small molecules which bind nucleic acids. As an example an antibiotic, e.g. edeine which specifically binds 16s RNA may be bound to the membrane. Another example of a capture probe may include an oligo dT probe suitable for capturing polyA RNA, e.g. mRNA. The membrane could be derivatized with a known ligand such as avidin or streptavidin or neutravidin. A biotinylated capture probe could be added to the membrane thereby conferring specificity to target nucleic acid sequence.

In certain embodiments the invention provides an automated system for isolating a nucleic acid from a sample. Thus any, or all of the steps may be automated, including applying the sample to the filter device, lysing the sample, washing the sample, eluting the sample. The automated system may comprise a computer, e.g. a personal computer programmed to carry out each of steps of the method described herein. The invention also contemplates processing multiple samples in a multiplex format.

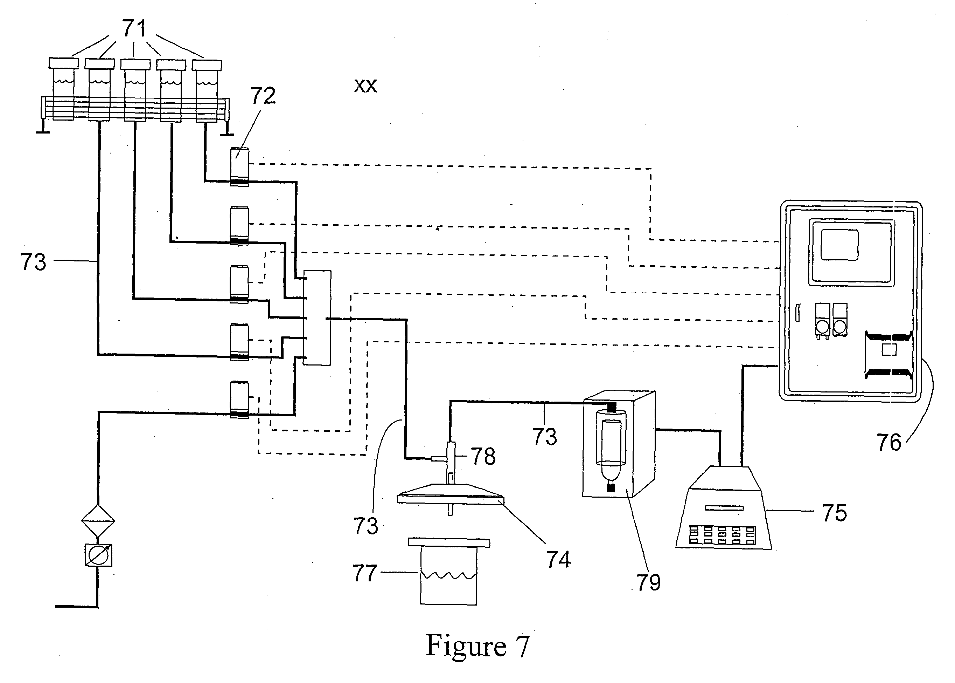

An example of an automated sytem is shown in Figure 7. thus certain embodiments the invention provide an automated system for isolating a nucleic acid from a sample comprising a) a filter device (74) suitable for isolating a nucleic acid from a sample; b) a pump (75), c) a program logic controller(PLC) (76), d) at least one container (71) suitable for holding a sample, e) optionally one or more containers (71) suitable for holding one or more reagents or buffers, f) optionally one or more receptacles suitable for receiving a filtrate from the filter device, an eluate from a filter device and/or a waste solution from a filter device (77); g) a plurality of pinch valves (72); h) tubing (73) and i) optionally a syringe cartridge positioned in a syringe barrel holder (79) which feeds one or more fluids from the tubing into the filter device (74). A check valve (78) is provided to allow application and/or removal of a fluid from the filter device.

The automated system according to the invention may be configured such that fluids including reagents, buffers, samples and the like never contact any moving parts, such as the moving parts of a pump. This may be achieved by using pinch valves which require no contact with a fluid passing through the system. The tubing and the filter device used in the system may suitable for a single use and thus may be disposable. The combination of disposable tubing and pinch valves provides a system which requires little maintenance e.g, cleaning between runs. Moreover because the tubing and filter device are used only once the risk of contamination of a sample is eliminated or minimized. This is particularly useful when the sample comprises a nucleic acid and downstream detection of the nucleic acid relies on an amplification protocol such as PCR or TMA. While providing superior sensitivity, amplification protocols also entail significant risk in amplifying a contaminating nucleic acid instead the target nucleic acid. The automated system described herein minimizes or eliminates such a risk and also provides an inexpensive system for performing nucleic acid isolation when compared to more traditional chromatography systems comprised of reusable (typically, metallic) parts where the fluids moving through the system contact the moving parts of the pump.

Detection of the isolated nucleic acid from the filtrate may be achieved using one or more amplification technologies such as polymerase chain reaction (PCR), reverse transcriptase polymerase chain reaction (rtPCR), real time PCR, and transcription mediated amplification (TMA). Detection of the nucleic acid may be achieved using gel electrophoresis and appropriate dye, e.g. a chemi-luminescent reagent, a fluorescent reagent.

### Filter Devices

The use of a variety of filtration device formats is contemplated. The filter may be a simple manual device or it may be a part of automated system. The size and the number of filtration devices may be dictated by the nature of sample and the target nucleic acid. In some embodiments, the filter device may be comprised of a solitary unit, e.g. a cartridge. The cartridge may comprise a housing, e.g. a hollow body for containing one or more membranes and one or more inlets and one or more outlets. The inlets and outlets may be sized for compatibility with a standard syringe or they may be sized to accommodate a vacuum source or a positive source of pressure. The cartridge may be sized to fit in a centrifuge. The filter device may be comprised of a column comprising a plurality of filters arranged sequentially within it such that a sample applied to the top of the column will contact each of the plurality of porous membranes within the column in a specified order. For example the first contacted membrane may have a nominal pore size greater than a subsequent contacted membrane. As another example the filter device may be comprised of one or more syringe cylinders which may be used to apply pressure or a vacuum to the device. The cartridge may be further comprised of a filtrate cup for collecting flow-through.

The filter device may be a multiplex e.g., comprised of multi-well plate, where at least one of the wells is comprised of a filter device comprising a plurality of membranes arranged sequentially such that a sample applied to the filter contacts a first membrane followed by one or more subsequent membranes, where the first membrane has a smaller nominal pore size than the subsequent membrane. Configuring all of the wells of a multi-well plate with the filter device described above is also contemplated. Suitable multi-well plates include plates comprised of a plurality of wells, e.g. 6 wells, 12 wells, 48 wells, 96 wells, 384 wells or more. The multi-well plates may be stacked one on top of another or may be used sequentially or separately. When used in a stacked configuration the stacked configuration may be disassembled such that target DNA and target RNA may be eluted from each of the respective membranes. Additionally, filter devices of the invention comprised of a single well plate are also contemplated.

In some embodiments a plate for receiving flow-through and waste material may be provided, i.e. a drain plate. The drain plate may be comprised of a single well or a plurality of wells depending on the particular analyte and sample source and whether or not subsequent analysis of the flow through is necessary. At least one of the plates, may be comprised of one or more inlets suitable for delivering a sample or a buffer solution to the filter device. At least one of the plates may be comprised of an outlet facilitating removal of flow-through and waste material. The outlet may be sized to accommodate a connection to a vacuum source. The multi-well plate may also be sized to fit in a centrifuge.

In one specific embodiment the filter device may be comprised of a mixed cellulose ester (MCE) membrane, e.g. having a nominal pore size of 0.45 microns overlaid over an APFF glass fiber membrane (Millipore Corp, Billerica, MA) having a nominal pore size of 0.7 microns. A space may exist between the MCE and APFF membranes thus facilitating isolation of both DNA and RNA. Alternatively the membranes may each be provided in their own housing or cartridge such that the mixed cellulose ester membrane is stacked on top of the APFF glass fiber memebrane. In other embodiments the invention provides for a polysaccharide membrane overlaid on a silica membrane configured in a suitable housing. For example a nitrocellulose membrane, overlaid on, or stacked over a glass fiber membrane is contemplated.

Where the filter device is configured as a cartridge the membranes may have a diameter of 13 mm. In other embodiments the membrane diameter may range from 1 mm up to 30 cms, from 1 mm up to 20 cm, from 0.1 mm up to 10 cm. In yet other embodiments the membrane diameter is less than 1 mm. In still other embodiments the membrane diameter is greater than 1 mm. In further embodiments the membranes may be configured in any shape for example the membrane could be configured as a square, a rectangle, a triangle, an ellipse or an irregular shape. The membrane may have a surface area ranging from 1 mm² to 30 cm². Alternatively, where the filter device is configured as a multi-well plate, the membranes may be sized to fit commercially available multiwell plates. A suitable multi-well plate may have at least one of the well comprised of a filter device comprising a plurality of membranes arranged sequentially such that a sample applied to the filter contacts a first membrane followed by one or more subsequent membranes, e.g., where the first membrane is comprised of a polysaccharide and the second membrane is comprised of silica.

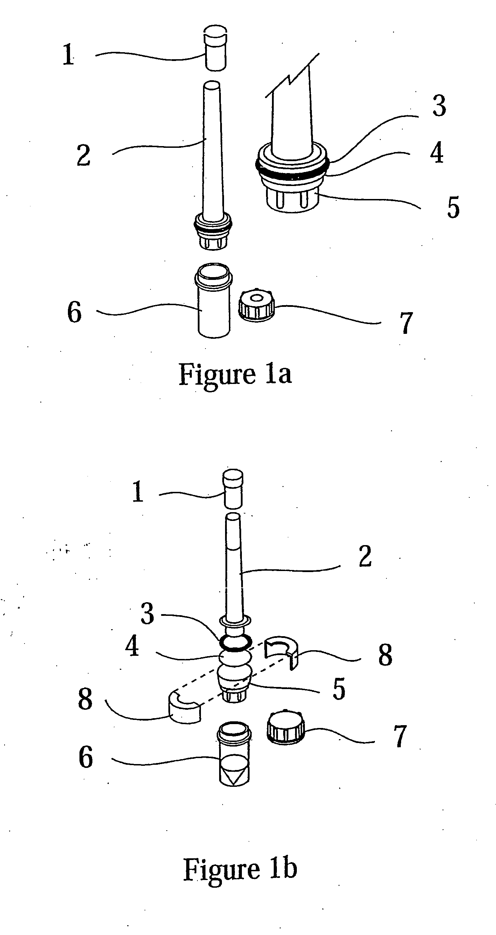

Figure 1 shows an example of a single use reusable filter device comprised of component parts according to the instant invention. The sample reservoir cap (1) is removed and the sample is applied to the sample reservoir (2) and the sample flows as a result of gravity or some external force applied to the device such that the sample contacts a first porous membrane of a plurality of porous membranes (4). The plurality of porous membranes may be seated on a membrane support (5) which is in fluid communication with a filtrate cup which may also serve as a receptacle for an eluted target. A filtrate cap (6) is also provided. Because the device may be disassembled and the plurality of porous membranes replaced, it is suitable for reuse. The membranes may be separated for elution and recapture of both DNA and RNA.

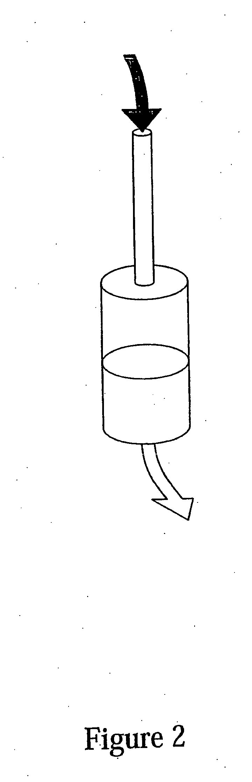

Figure 2 shows an example of single use filtration device of the instant invention which is not re-useable. The single use filtration device depicted may be used in series to purify both DNA and RNA from a sample such as a cell lysate.

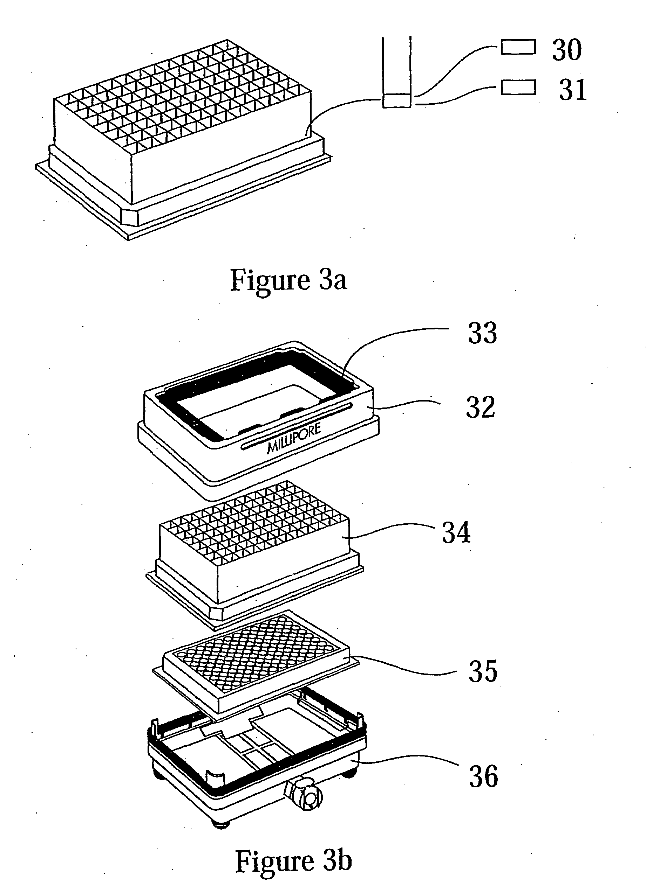

Figure 3a shows an example of a multiwell filtration device according to the present invention. Each well comprises a porous membrane (30). A plurality of plates may be stacked one upon the other. Each plate may be comprised of a different porous material. Thus in one embodiment a plate comprising a polysaccharide membrane may be stacked on top of a plate comprising a silicate membrane. The stacked plates may be unstacked to elute and recapture isolated RNA and DNA.

Figure 3b shows another example of an apparatus comprising a multiwell filtration device according to the present invention. The apparatus may include a removable collar (32), which comprises a collar gasket seated within the collar (33) to provide a seal to facilitate the application of a vaccuum. The collar (32) engages one or more filter plates (34) comprising a plurality of wells, wherein at least one of the wells is comprised of a porous membrane. Where a plurality of stacked plates is used, each comprised of a porous membrane, the first porous membrane may be a polysaccharide membrane such as a mixed cellulose ester membrane or a nitrocellulose membrane and the second porous membrane may be a silicate membrane, such as a glass fiber membrane. The porous membranes may be stacked one upon the other. The filter plates may be separated after being contacted with the sample to provide for separate elution and recapture of the DNA, the RNA or both. Each filter plate (34) may be seated on a collection plate (35) which is comprised of a plurality of wells suitable for receiving an isolated nucleic acid according to the methods of the invention. The collection plate wells may correspond one to one with the wells of the filter plate. The collection plate may be seated on a base (36) which may comprise a vacuum manifold.

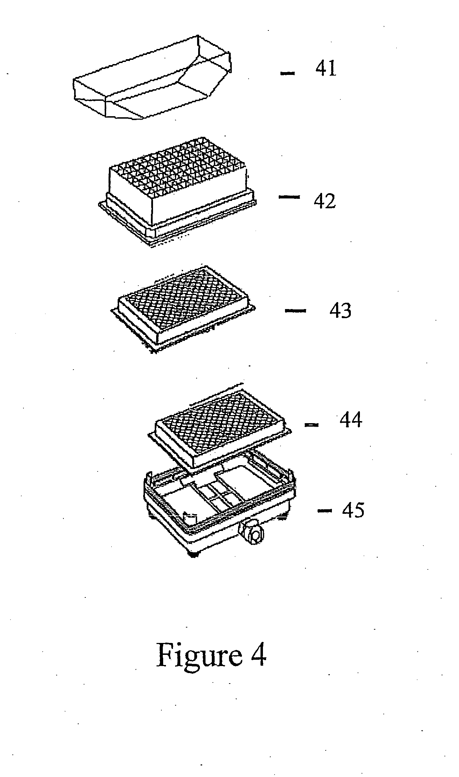

Figure 4 shows another example of a multiwell filtration device according to the present invention. The device may comprise a hopper (41) for receiving a sample which contacts a removable filter plate comprised of a plurality of wells for receiving samples, e.g. large volume samples, where at least one of the wells is comprised of one or more porous membranes (42). In some embodiments two filter plates (42) may be stacked upon each other. The first filter plate may comprise a polysaccharide membrane and the second plate may comprise a silicate membrane. One or more of the filter plates may be seated on an additional multiwell plate e.g., Ziptip® (Millipore Corp., Billerica, MA)(43). The wells of the Ziptip® plate may correspond one to one with the wells of the filter plate. The Ziptip® may comprise a resin or other solid support suitable for retaining a nucleic acid, such as RNA. In specific embodiments the resin may be comprised of poly A, poly U, or poly T sepharose. The Ziptip® plate may be seated on a collection plate comprised of a plurality of wells (44). The wells may correspond one to one with the wells of the Ziptip® plate and may contain TMA reagents such as reconstituted amplification reagent with amplification oligos reconstituted enzyme. The collection plate may be seated in a base comprising a multiscreen vacuum manifold (45).

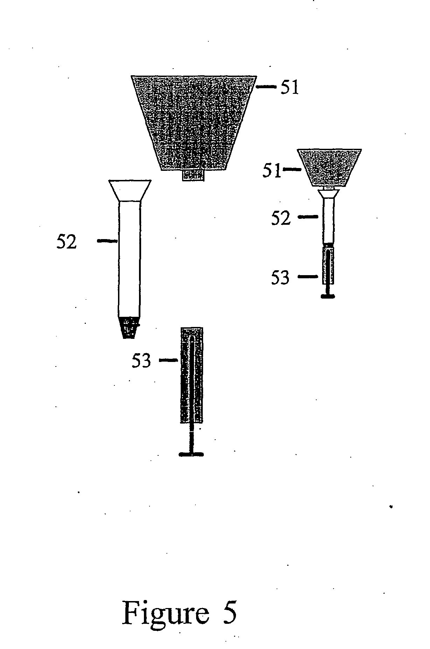

Figure 5 shows another example of a single sample filtration device according to the instant invention. Two or more of the devices may be used in series for isolating both DNA and RNA from a sample, each device being comprised of a resin, e.g. a membrane, suitable for binding DNA or RNA. The device may comprise a receptacle such as Microfil/Milliflex® (51) (Millipore Corporation, Billerica, MA). The receptacle may be seated on a micro-column loaded with nucleic acid capture resin suitable for retaining a target nucleic acid analyte (52). The resin may be suitable for retaining a target analyte such as a nucleic acid from a sample, e.g. a cellular lysate comprising RNA and DNA, e.g., genomic DNA. The resin may comprise a polysaccharide such as cellulose. The resin may comprise a silicate such as glass fiber. The micro column may be in fluid communication with a syringe (53) suitable for pulling lysate through the column.

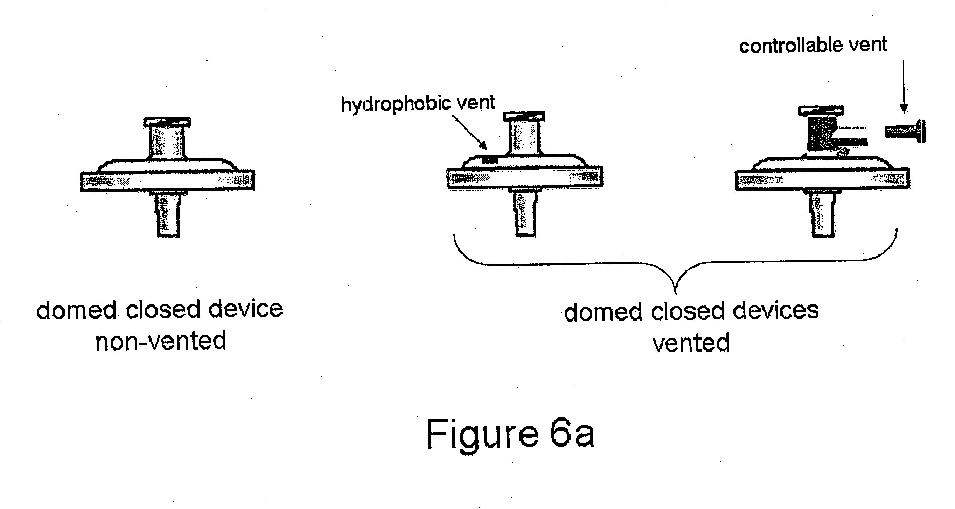

Figure 6a shows three examples of sealed closed dome filtration devices. The closed dome device may provide for processing a sample under sterile conditions. The dome may serve as a housing for one or more porous membranes and may further comprise one or more vents, which do not serve as inlets for samples or reagents. The vents may be comprised of any suitable material which allows for the passage of air and other gases but prevents particulate matter such as microbes from passing. In one embodiment the vent is comprised of a gas accessible hydrophobic barrier. A hydrophillic barrier is also contemplated. In some embodiments the vent may be positioned on a surface of the domed housing structure. In another embodiment the vent may be positioned as a side arm leading into a portion of the domed housing such as an inlet in communication with the portion of the domed housing containing the one or more porous membranes. When a sample is flowing through the filter device the vent may be kept closed by using a luer-locked plug. The plug may be comprised of solid material, or may itself be vented and thus comprised of a membrane barrier such as a hydrophobic membrane. The use of vents provides a means of relieving pressure and thus preventing damage to the filter.

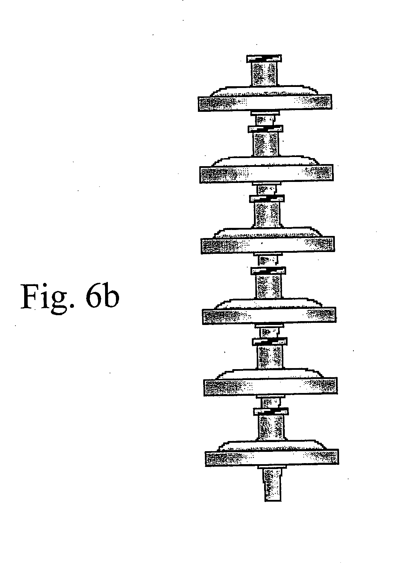

Figure 6b shows an example of a single use membrane device according to the invention where the device comprises a plurality of stacked membrane cartridges. Each cartridge in the device is in fluid communication with the next adjacent cartridge. The cartridge may be comprised of one or more porous membranes. Where the cartridge comprises more than one porous membrane, the membranes may be the same or different from the other porous membranes contained within the cartridge. In some embodiments the multiple membranes within a cartridge may be fused using heated polypropylene. As an example the first cartridge may be comprised of a mixed cellulose ester (MCE) membrane and the second cartridge stacked beneath the first may be comprised of a glass fiber membrane. The MCE may have a nominal pore size that is smaller than the glass fiber filter.

Figure 7 shows an example of a multiple membrane vented device according to the invention. The device may be connected by two-way vacuum valve (check valve assembly) to a syringe. The 2-way vacuum valve may be plumbed to a solution manifold. From the solution manifold emanate multiple lines connected to solution containers (i.e. sample, lysis, wash, elution solutions/buffers) and an air vent. Each solution line may be controlled by an electrical pinch valve. Sample, followed lysis buffer, followed by washes, followed by elution buffer may each be sequentially introduced in order to capture and lyse cells on a surface of the first porous membrane in the device and then capture, wash, and elute nucleic acid from the stacked membranes in the device. The first porous membrane of the device may be comprised of a polymer such as MCE. The second may be comprised of silica such as glass fiber. The nominal pore size of the polymer membrane may be smaller than the nominal pore size of the silica membrane. The system shown in Figure 7 is one example of how automated can be accomplished.

### Membranes

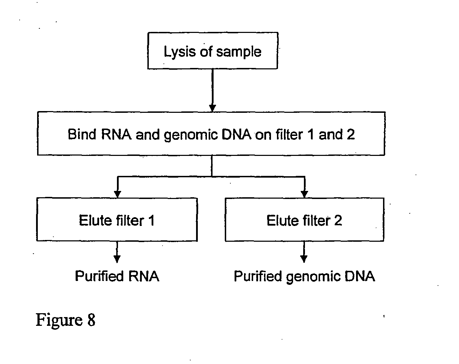

Membranes may be arranged in a stacked configuration or in series. (Figure 8). Membranes used in the invention may be comprised of any porous material known in the art. Examples of suitable porous materials, include, but are not limited to polyether sulfone, polyamide, e.g., agarose, cellulose, a polysaccharide, polytetrafluoroethylene, polysulfone, polyester, polyvinylidene fluoride, polypropylene, a fluorocarbon, e.g. poly (tetrafluoroethylene-co-perfluoro(alkyl vinyl ether)), poly carbonate, polyethylene, glass, polycarbonate, ceramic, nylon, carbon nano-tube and metal.

In certain specific embodiments the first membrane may be comprised of mixed cellulose. In other specific embodiments the first membrane may be comprised of nitrocellulose. The membrane may comprise nitrocellulose in an amount ranging from 0.1% to 100%; 1.0%-99.9%; 5%-95%; 10%-90%; 20%-80%; 30%-70%; 40%-60%. In some embodiments the first membrane may be comprised of at least 50% nitrocellulose.

The second membrane may be comprised of glass or glass fibers. In some embodiments an additional resin suitable for retaining nucleic acid, e.g., RNA may be used. If an optional third membrane comprising a capture probe is desired, the third membrane may be comprised of any known porous material, e.g., nylon, polyethylene sulfone.

A suitable membrane according to the invention may have a pore size ranging from 0.0001 micron to 100 microns, from .1 micron to 80 microns, from 1 micron to 50 microns, from 2 microns to 45 microns. In some embodiments the pore size of the top membrane, or the membrane which contacts the sample first may be smaller than the pore size of the second or subsequent membrane. In specific embodiments at least one of the membrane has a pore size of at least 2 microns. In other embodiments at least one of the membranes has a pore size of at least 45 microns. In certain embodiments the invention provides for a cellulose membrane having a pore size ranging from 0.1 micron to 50 microns, e.g. 45 microns. In specific embodiments the invention provides for a silicate membrane having a pre size ranging from 0.1 micron to 100 microns. In other embodiments the invention provides for a silicate membrane having a pore size of 0.7 microns.

A significant advantage of using membranes over porous beads for analyte absorption or chromatography is the difference in liquid flow patterns between the two formats. When using porous beads such as in packed beds, an analyte in an applied convective flow diffuses to the film surface and also then slowly diffuses within the pores of the bead. When using a membrane, an analyte in an applied convective flow quickly moves though a membrane and only needs to diffuse to the film surface. Saturation binding of an analyte can potentially be more quickly achieved using membranes than beads. By membrane what is meant is a structure, having lateral dimensions much greater than its longitudinal dimensions, through which mass transfer may occur under a variety of driving forces.

In addition to normal flow devices, in which bulk convection is perpendicular to the membrane surface, radial flow devices can also be used for analyte absorption and chromatography. In radial flow devices, flow is through a membrane wrapped around porous cylindrical frits. The flow can occur from inside out or outside in.

### Nucleic Acid Samples

Sample, as used herein, refers to material comprising nucleic acid derived from any biological source. The biological source may be any living or dead thing such as a plant, an animal, a viral particle. The biological source may be a whole organism or an organ or tissue derived from an organism. The biological source may be unicellular or multicellular, or non-cellular, e.g. viral. Cellular sources may include eukaryotic cells, prokaryotic cells including mycoplasma. Examples of eukaryotic cells may include cells derived from any mammal, e.g., humans, non-human primates, horses, goats, sheep, rats, rabbits, mice, guinea pigs. Examples of prokaryotic cells include gram negative bacteria and gram positive bacteria such as *Escherichia coli, Pseudomonas aeruginosa, Staphylococcal aureus.* Where the samples are cellular samples, the cells may be lysed before being contacted with the plurality of membranes. In the case where the cellular sample is a prokaryotic sample, the sample may be clarified, by centrifugation for example, before being contacted with the plurality of membranes. The sample may include naturally occurring samples or those created or manipulated either partially or wholly by the human hand.

Target nucleic acids for isolation according to the methods disclosed herein include any nucleic acid found in a sample including DNA, RNA, PNA, LNA, and hybrids of more than one type of nucleic acid. The nucleic acid may be double stranded, single stranded, or multi-stranded. Where the target is DNA, the DNA may be plasmid DNA, vector DNA, genomic DNA, including mitochodrial DNA, or a fragment of any of the preceding. Where the target is comprised of RNA the RNA may be mRNA, tRNA or rRNA e.g. 16s RNA, 23s RNA. The nucleic acid may be comprised of nucleotide analogs, e.g., chain terminators. The size of the target nucleic acid may range from 2-30 nucleotides or may be in kilobase or larger range.

### Kits

The invention also provides for kits which may be used to isolate nucleic acids from a sample. The kit may comprise one or more filtration devices according to the instant invention and one or more containers. The kit may contain one or more controls or sample target analytes or specimens. The kit may optionally include various buffers useful in the methods of the invention. As an example the kit may include a lysis buffer suitable for lysing cells when the target nucleic acid is contained in a sample comprised of cells. The kit may also optionally include wash buffers for eliminating reagents or non-specifically retained or bound material. The kit may also optionally include an elution buffer for eluting a bound target nucleic acid from a membrane. Each of the buffers may be provided in a separate container as a solution. Alternatively the buffers may be provided in dry form or as a powder and may be made up as a solution according to the user's desired application. In this case the buffers may be provided in packets. The kit may provide a power source in instances where the device is automated. The kit may also comprise a vacuum pump. The kit may also include instructions for using the device and for making up reagents suitable for use with the device and methods according to the instant invention. The kit may optionally include software for recording and analyzing data obtained while practicing the methods of the invention or while using the device of the invention.

### Examples

### Example 1: Simultaneous RNA and genomic DNA purification using centrifugal devices

An overnight culture of Pseudomonas Pseudoalcaligenes was harvested by centrifugation. The bacterial pellet was resuspended in Tris-EDTA (TE) buffer with lysozyme (1 mg/ml). After an incubation of 5 minutes, lysis buffer (3 M GuSCN, 0.01 M TRIS-HCI pH 7.6, 0.035 M EDTA) with 1% β-mercaptoethanol was added and the bacterial lysate was centrifuged. The supernatant was transferred to a new tube and 100% ethanol was added to the lysate.

The clarified lysate mixture, comprising 1×10⁹ bacteria, was loaded onto centrifugal devices comprising a single MCE membrane (Millipore Corporation, Billerica, MA). The device was centrifuged and the filtrate was loaded onto a centrifugal device with a single glass fiber membrane with a nominal pore size of 0.7 microns (APFF) (Millipore Corporation, Billerica, MA). The latter device was centrifuged and the filtrate was discarded. Both the MCE membrane and glass fiber membrane device were washed with wash buffer 1 comprising: 1 M GuSCN, 0.01 M TRIS-HCl pH 7.6, 0.035 M EDTA, 25% EtOH, followed by two washes with wash buffer 2 comprising: 70 % EtOH in 0.01 M TRIS-HCl pH 7.6.

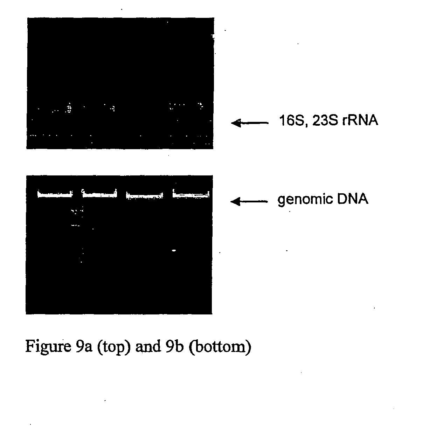

Subsequently, an elution step using water as the elution buffer was carried out on the MCE filter device. The eluate contained isolated RNA (see Figure 9a). Elution with water of the glass fiber membrane resulted in isolated genomic DNA in the eluate (Figure 9b).

### Example 2: Simultaneous RNA and genomic DNA purification from prokaryotic cells using stacked filter plates

An overnight culture of Pseudomonas Pseudoalcaligenes was harvested by centrifugation. The bacterial pellet was resuspended in TE buffer with lysozyme (1 mg/ml). After an incubation of 5 minutes, lysis buffer (as described in Example 1) with 1% β-mercaptoethanol was added and the bacterial lysate was centrifuged. The clarified supernatant was transferred to a new tube and ethanol was added to a final concentration of 35% to the lysate.

This mixture was loaded onto a stack of 96-well filter plates with a MCE filter plate on top and a glass fiber filter plate on the bottom so that the mixture contacted the MCE filter plate first. The clarified supernatant comprising 1 to 5 ×10⁸ lysed bacteria was loaded in each well. The stack of filter plates was centrifuged and the filtrate was discarded. Wash buffer 1 (as described in Example 1) was added to each well and plates were centrifuged, now as single plates. Two more washes were performed with wash buffer 2 (as described in Example 1).

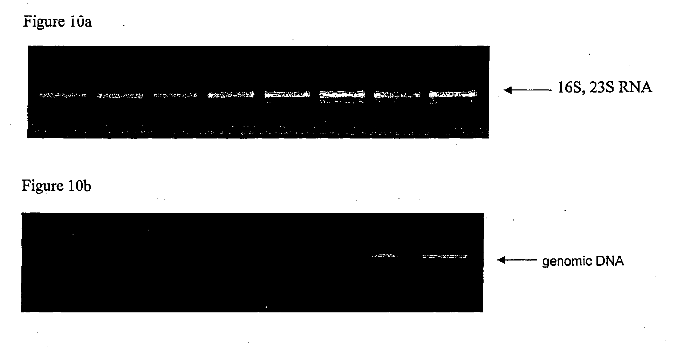

Cellular RNA and genomic DNA were eluted with water from respectively the MCE filter plate (Figure 10a) and glass filter plate (Figure 10b).

### Example 3: Simultaneous RNA and genomic DNA purification from eukaryotic cells using stacked filter plates

3T3 NIH fibroblasts were trypsinized and pelleted. The pellet was resuspended in lysis buffer (as described in Example 1) with 1 % β-mercaptoethanol. ETOH was added to a final concentration of 35% to the lysate and the mixture was loaded onto the MCE and glass fiber filter plate stack. 5×10⁵ fibroblasts were loaded per well. The stack of filter plates was centrifuged and the filtrate was discarded. Wash buffer 1(as described in Example 1) was added to each well and the plates were centrifuged, now as single plates. Two more washes were performed with wash buffer 2 (as described in Example 1).

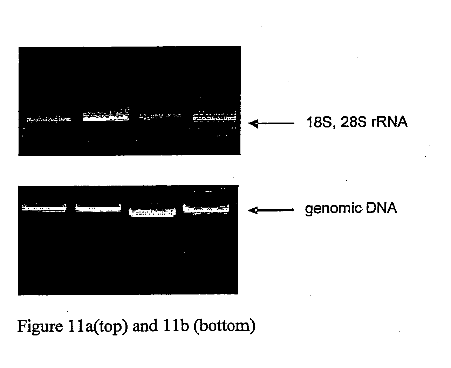

Cellular RNA and genomic DNA were eluted with water from respectively the MCE filter plate (Figure 11 a) and glass filter plate (Figure 11b).

### Example 4: Qualitative assessment of RNA by RT-PCR

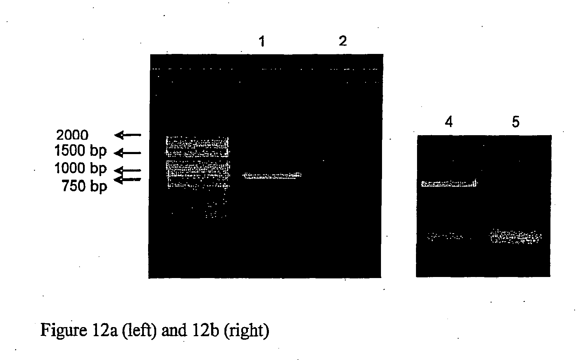

Prokaryotic as well as eukaryotic RNA (200 ng) eluted from a single well from the MCE filters used in the experiments described above was transcribed to cDNA using the Protoscript First Strand cDNA Synthesis Kit (New England BioLabs, Beverly, MA)(NEB). cDNA was synthesized with random primers and according to the NEB kit's manual. For each cDNA synthesis a negative control, sample without reverse transcriptase, was included (Figure 12 lane 2 and 5). The band appearing in lane 5 is primer dimers.

Eukaryotic cDNA was amplified by standard methods using β-actin primers (forward: GTG GGG CGC CCC AGG CAC CA (SEQ ID NO: 1), reversed: CTC CTT AAT GTC ACG CAC GAT, (SEQ ID NO: 2) (Applequist et al. 2002, International Immunology 14(9): 1065-1074), see figure 12a. Prokaryotic cDNA was amplified by PCR using 16S rRNA genes primers (forward Ps-for: GGT CTG AGA GGA TGA TCA GT, (SEQ ID NO: 3) reversed Ps-rev TTA GCT CCA CCT CGC GGC), (SEQ ID NO: 4) (Widmer et al. 1998, *Applied and Environmental Microbiology,* 64(7):2545) see figure 12b.

### Example 5: Comparison of RNA purity using either a single MCE filter in a centrifugal device, or a commercially available column

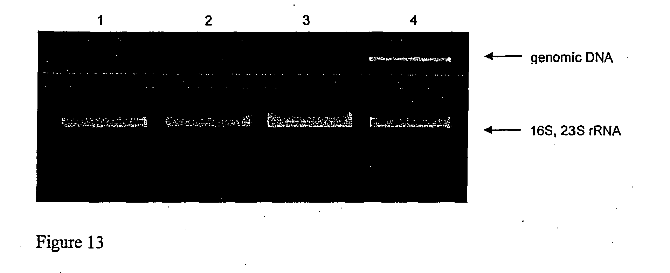

An overnight culture of *Pseudomonas Pseudoalcaligenes* was harvested by centrifugation. The bacterial pellet was resuspended in TE buffer with lysozyme (1 mg/ml). After an incubation of 5 minutes, lysis buffer with 1% β-mercaptoethanol was added and the bacterial lysate was centrifuged. The clarified supernatant was transferred to a new tube and 100% ethanol was added to the lysate. The mixture was loaded onto three centrifugal devices with a single MCE membrane and onto one Midi RNeasy® spin column (Qiagen, Valencia, CA). Clarified supernatant from 1 x10⁹ bacteria per device was loaded on either the membrane device or the column. The devices and the column were centrifuged and filtrate discarded. They were washed subsequently with wash buffer 1 (as described in Example 1) and twice with wash buffer 2 (as described in Example 1). RNA was eluted with water. Figure 13 shows that the purification with MCE membrane devices leads to pure RNA. The Qiagen RNeasy® column device leads to an RNA product still contaminated with a significant amount of genomic DNA (Figure 13, lane 4).

All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit of the invention being indicated by the following claims.

## Claims

1. A method of isolating DNA and RNA from a cellular sample comprising a) lysing the cellular sample with a lysis buffer; b) optionally clarifying the cellular sample to obtain a clarified supernatant; c) contacting a first membrane comprising a polysaccharide with the lysate, such that the RNA binds to the first membrane; and d) contacting the lysate, or a filtrate of the first membrane, with a second membrane, such that the DNA binds to the second membrane thereby isolating DNA and cellular RNA from the cellular sample.

2. The method of claim 1, wherein:
a) the DNA is genomic DNA; and/or
b) the lysis buffer has a non-acidic pH, and/or
c) the lysis buffer comprises a chaotropic agent and a chelating agent; and/or
d) the clarifying step comprises centrifuging the sample; and/or
e) the first membrane is a mixed cellulose ester membrane; and/or
f) the second membrane is a silicate membrane.

3. The method of claim 1 or claim 2, further comprising washing the first and second membranes with a plurality of wash buffers; and optionally, wherein one or more of:
a) the plurality of wash buffers has a non-acidic pH; and/or
b) the plurality of wash buffers includes one buffer comprised of a chaotropic agent, a chelating agent and an alcohol; and/or
c) the plurality of buffers includes at least one buffer comprised of an alcohol; and/or
d) the membranes are washed first with a buffer comprising a chaotropic agent, a chelating agent and an alcohol followed by a wash buffer comprising an alcohol.

4. The method of any one of claims 1 to 3, further comprising contacting the first and second membrane with an elution buffer; and in which case optionally, wherein the elution buffer is water.

5. The method of any one of claims 1 to 4, wherein the first and second membranes each comprise a multiwell plate or a single well plate; and in which case optionally, wherein one or more of:
a) the multiwell plates or the single well plates are in a stacked configuration; and/or
b) the multiwell plates or the single well plates are centrifuged after step c); and/or
c) a vacuum is applied to the multiwell plates or the single well plates after step c).

6. A method of isolating genomic DNA and cellular RNA from a cellular sample comprising a) lysing the cellular sample with a lysis buffer having a pH of 7.6 and comprising 3 molar guanididium thio-cyanate, 0.01 molar TRIS-HCl, and 0.035 molar EDTA; b) centrifuging the cellular sample to obtain a clarified supernatant; c) contacting the supernatant with a mixed cellulose ester membrane, such that the RNA binds to the membrane; and d) contacting a second membrane with the supernatant or a filtrate of the first membrane, such that the DNA binds to the second membrane comprising glass fiber; e) washing both membranes with a non-acidic wash buffer comprising a chaotropic agent, a chelating agent and an alcohol; f) washing both membranes with a non-acidic wash buffer comprising an alcohol thereby isolating DNA and cellular RNA from the cellular sample.

7. The method of claim 6, further comprising eluting at least one of the DNA and RNA from its respective membrane with water.

8. A kit for isolating DNA and RNA from a cellular sample comprising a) a first membrane comprised of a polysaccharide and second membrane comprised of silica; b) optionally one or more non-acidic wash buffers; c) one or more containers.

9. The kit of claim 8, wherein the first membrane is mixed cellulose ester membrane and the second membrane is a glass fiber membrane.

10. A kit for isolating RNA from a cellular sample comprising a) a mixed cellulose ester membrane; b) one or more non-acidic wash buffers; and c) one or more containers.

11. The kit of one of any one of claims 8 to 10, wherein:
a) the one or more wash buffers comprise a first non-acidic wash buffer comprising a chaotropic agent, a chelating agent and an alcohol and a second non acidic wash buffer comprises an alcohol; and/or
b) the kit further comprising a non-acidic lysis buffer suitable for lysing a cellular sample; and/or
c) the lysis buffer comprises a chaotropic agent and a chelating agent.
